# EUROPÄISCHE PATENTANMELDUNG

(11) **EP 0 530 702 A1**
(43) Veröffentlichungstag der Anmeldung: **10.03.1993**
(21) Anmeldenummer: 92114738.5
(22) Anmeldetag: 28.08.1992
(51) Int. Cl.: C07D 213/64, C07D 213/80, C07D 213/82, C07D 213/85, C07D 401/10, C07D 401/14, A61K 31/44

(54) **1,2-Dihydro-2-oxopyridine als Angiotensin II-Antagonisten**

(30) Priorität: 04.09.1991 DE 4129340
(71) Anmelder: MERCK PATENT GmbH, D-64271 Darmstadt (DE)
(72) Erfinder: Mederski, Werner, Dr., W-6106 Erzhausen (DE); Beier, Norbert, Dr., W-6107 Reinheim 5 (DE); Schelling, Pierre, Dr., W-6109 Mühltal (DE); Lues, Ingeborg, Dr., W-6100 Darmstadt (DE); Minck, Klaus-Otto, Dr., W-6105 Ober-Ramstadt (DE)

(57) **Zusammenfassung**

Neue 1,2-Dihydro-2-oxo-pyridine der Formel I 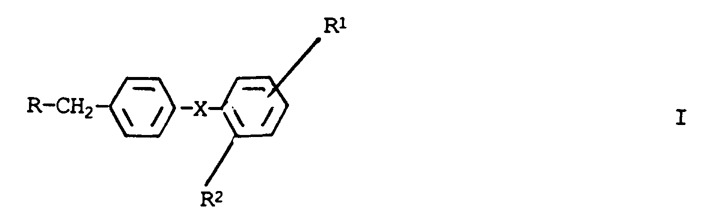
worin
- R: den Rest 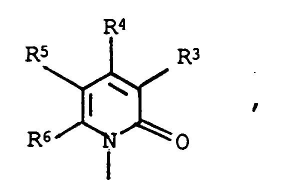
bedeutet und
- R¹ bis R⁶ und X: die in Patentanspruch 1 angegebene Bedeutung haben,
sowie deren Salze zeigen angiotensin II-antagonistische Eigenschaften und können zur Behandlung von Hypertension, Aldosteronismus und Herzinsuffizienz verwendet werden.

## Beschreibung

Die Erfindung betrifft neue 1,2-Dihydro-2-oxopyridine der Formel I 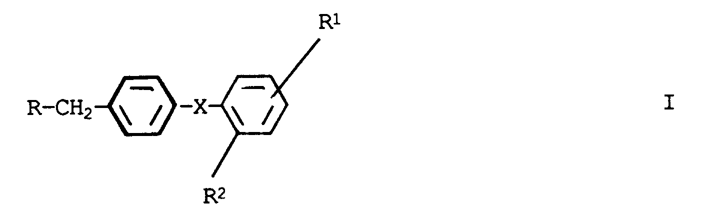
worin
- R: den Rest 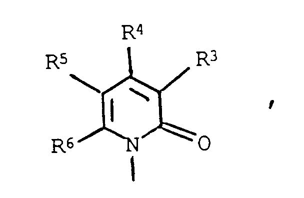
- R¹: H, Hal, A, OA oder NO₂,
- R²: COOH, COOA, CN, NO₂, NH₂, NHCOR⁷, NHSO₂R⁷ oder 5-Tetrazolyl,
- R³: H, COOH, COOA, CHO, CN, NO₂, CH₂R⁸, CH₂OR⁹, NR¹⁰R¹¹, CH₂NR¹⁰R¹¹, CONR¹⁰R¹¹ oder 5-Tetrazolyl,
- R⁴: H oder A,
- R⁵, R⁶ und R¹⁰: jeweils H, A, Alkenyl mit 2-6 C-Atomen, Alkinyl mit 2-6 C-Atomen, Ar oder Ar-alkyl mit 1-6 C-Atomen im "alkyl"-Teil,
- R⁷: A oder ein- oder mehrfach durch F substituiertes A,
- R⁸: H, Hal, A, Ar, CN, COOH, COOA, CH₂COOH, CH₂COOA oder 5-Tetrazolyl,
- R⁹: H, A, Ar, Ar-alkyl mit 1-6 C-Atomen im "alkyl"-Teil, COA, COAr, COOA, COOAr, CONR¹²R¹³, COO-alkyl-Ar oder A-O-alkyl mit jeweils 1-6 C-Atomen im "alkyl"-Teil,
- R¹¹: H, A, ein- oder mehrfach durch F substituiertes A, Ar, Ar-alkyl mit 1-6 C-Atomen im "alkyl"-Teil, CO-A, CO-Ar, CO-alkyl-Ar mit 1-6 C-Atomen im "alkyl"-Teil, COOA, COOAr, COO-alkyl-Ar mit 1-6 C-Atomen im "alkyl"-Teil, CONR¹²R¹³, SO₂R⁷ oder SO₂Ar,
- NR¹⁰R¹¹: auch Pyrrolidino, Piperidino, Morpholino, Succinimido oder Phthalimido,
- R¹² und R¹³: jeweils H, A, Cycloalkyl mit 3-8 C-Atomen, Alkenyl mit 2-6 C-Atomen, Alkinyl mit 2-6 C-Atomen oder Ar,
- X: fehlt, -CO-, -O-, -NH-CO-, -CO-NH-, -CH₂-O- oder -O-CH₂-,
- A: Alkyl mit 1-6 C-Atomen,
- Ar: unsubstituiertes oder einfach durch A, OA, CF₃, Hai oder NO₂ substituiertes Phenyl und
- Hal: F, Cl, Br oder I bedeuten,
worin jedoch mindestens eine der folgenden Bedingungen erfüllt sein muß:
(a) R¹ bedeutet Hal, A, OA oder NO₂,
(b) R² bedeutet CN, NO₂, NH₂, NHCOR⁷ oder NHSO₂R⁷,
(c) R³ bedeutet CH₂Ar, CH₂CN, CH₂COOH, CH₂COOA, CH₂CH₂COOH, CH₂CH₂COOA, CH₂-(5-Tetrazolyl); CH₂OAr, CH₂O-alkyl-Ar' (worin Ar' eine einfach durch A, OA, CF₃, Hal oder NO₂ substituierte Phenylgruppe bedeutet), CH₂O-CO-Ar', CH₂OCOOAr, CH₂OCONR¹⁴R¹⁵ (worin R¹⁴ und R¹⁵ jeweils unabhängig voneinander Cycloalkyl, Alkenyl, Alkinyl oder Ar bedeuten, einer der Reste R¹⁴ und R¹⁵ auch H oder A bedeuten kann), CH₂O-COO-alkyl-Ar, CH₂O-alkyl-O-A; NR¹⁶R¹⁷ (worin R¹⁶ Alkenyl, Alkinyl, Ar oder Ar-alkyl, R¹⁷ ein- oder mehrfach durch F substituiertes A, Ar, Ar-alkyl, CO-Ar, CO-alkyl-Ar, COOA, COOAr, COO-alkyl-Ar oder CONR¹²R¹³ bedeuten, einer der Reste R¹⁶ und R¹⁷ auch H oder A, die Gruppe NR¹⁶R¹⁷ auch Pyrrolidino, Piperidino, Morpholino, Succinimido oder Phthalimido bedeuten kann); CH₂NR¹⁰R¹¹; CONR¹⁸R¹⁹ (worin R¹⁸ Alkenyl, Alkinyl, Ar oder Ar-alkyl, R¹⁹ ein- oder mehrfach durch F substituiertes A, Ar', Ar-alkyl, CO-Ar, CO-alkyl-Ar, COOA, COOAr, COO-alkyl-Ar oder CONR¹²R¹³ bedeuten, einer der Reste R¹⁸ und R¹⁹ auch H oder A, die Gruppe NR¹⁸R¹⁹ auch Succinimido oder Phthalimido bedeuten kann);
(d) R⁵ bedeutet Alkinyl, Ar' oder Ar-alkyl,
(e) R⁶ bedeutet Alkinyl, Ar' oder Ar-alkyl,
(f) X bedeutet -CO-, -O-, -NH-CO-, -CO-NH-, -CH₂-O- oder -O-CH₂-,
sowie ihre Salze.

Ähnliche Verbindungen sind aus EP-A2-400974, US-A1 4 880 804 und WO 91/19697 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie angiotensin II-antagonistische Eigenschaften und können daher zur Behandlung der angiotensin II-abhängigen Hypertension, des Aldosteronismus und der Herzinsuffizienz eingesetzt werden. Diese Wirkungen können nach üblichen in vitro- oder in vivo-Methoden ermittelt werden, wie sie z.B. in der US-PS 4 880 804 beschrieben sind, ferner von A.T. Chiu et al., J. Pharmacol. Exp. Therap. 250, 867-874 (1989), und von P.C. Wong et al., ibid. 252, 719-725 (1990; in vivo, an Ratten).

Die Verbindungen der Formel I können als Arzneimittelwirkstoffe in der Human- und Veterinärmedizin eingesetzt werden, insbesondere zur Prophylaxe und/oder Therapie von Herz-, Kreislauf- und Gefäßkrankheiten, vor allem von Hypertonie, Herzinsuffizienz und Hyperaldosteronismus.

Gegenstand der Erfindung sind die Verbindungen der Formel I und ihre Salze sowie ein Verfahren zur Herstellung dieser Verbindungen sowie ihrer Salze, dadurch gekennzeichnet, daß man
(a) eine Verbindung der Formel II 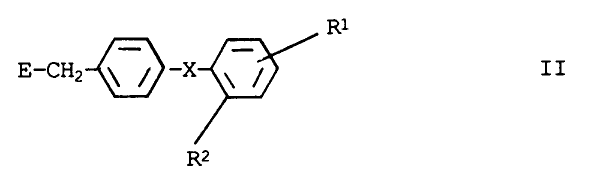 worin
   - E: Cl, Br, I oder eine freie oder reaktionsfähig funktionell abgewandelte OH-Gruppe bedeutet und
   - R¹, R² und X: die in Anspruch 1 angegebene Bedeutung haben, mit einer Verbindung der Formel III

   H-R III

   worin
   - R: die in Anspruch 1 angegebene Bedeutung hat,
   oder
b) zur Herstellung einer Verbindung der Formel I, worin X -NH-CO- oder -CO-NH- bedeutet, eine Verbindung der Formel IV 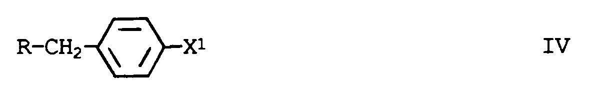 worin
   - X¹: NH₂ oder COOH bedeutet und
   - R: die in Anspruch 1 angegebene Bedeutung hat
   oder ein reaktionsfähiges Derivat dieser Verbindung mit einer Verbindung der Formel V 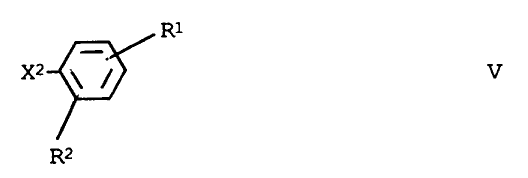 worin
   - X²: COOH (falls X¹ NH₂ ist) oder NH₂ (falls X¹ COOH ist) bedeutet und
   - R¹ und R²: die in Anspruch 1 angegebenen Bedeutungen haben
   oder mit einem reaktionsfähigen Derivat dieser Verbindung oder
(c) zur Herstellung einer Verbindung der Formel I, worin X -CH₂-O- oder -O-CH₂- bedeutet, eine Verbindung der Formel VI 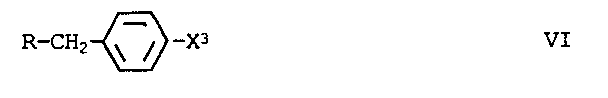 worin
   - X³: CH₂E oder OH bedeutet und
   - R: die in Anspruch 1 angegebene Bedeutung hat
   oder ein reaktionsfähiges Derivat dieser Verbindung mit einer Verbindung der Formel VII 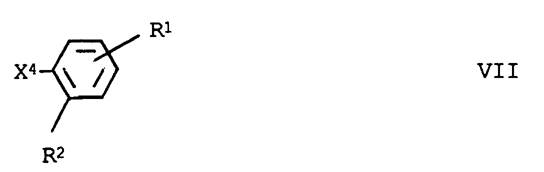 worin
   - X⁴: OH (falls X³ CH₂E ist) oder CH₂E (falls X³ OH ist) bedeutet und
   - R¹ und R²: die in Anspruch 1 angegebenen Bedeutungen haben
   oder mit einem reaktionsfähigen Derivat dieser Verbindung
umsetzt,
oder daß man eine Verbindung der Formel I aus einem ihrer funktionellen Derivate durch Behandeln mit einem solvolysierenden oder hydrogenolysierenden Mittel in Freiheit setzt,
und/oder daß man in einer Verbindung der Formel I einen oder mehrere Rest(e) R¹, R², R³, R⁴, R⁵ und/oder R⁶ in einen oder mehrere andere Reste R¹, R², R³, R⁴, R⁵ und/oder R⁶ umwandelt und/oder eine Base oder Säure der Formel I in eines ihrer Salze umwandelt.

Vor- und nachstehend haben die Reste bzw. Parameter R, R¹ bis R¹³, X, A, Ar, Hal, E, X¹, X², X³ und X⁴ die bei den Formeln I bis VII angegebenen Bedeutungen, falls nicht ausdrücklich etwas anderes angegeben ist.

In den vorstehenden Formeln hat A 1-6, vorzugsweise 1, 2, 3, oder 4 C-Atome. A bedeutet vorzugsweise Methyl, Ethyl, Propyl, Isopropyl, Butyl, Isobutyl, sek.-Butyl, oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl.

Ar ist vorzugsweise unsubstituiertes Phenyl, ferner bevorzugt o-, m- oder p-Tolyl, o-, m- oder p-Ethylphenyl, o-, m- oder p-Isopropylphenyl, o-, m- oder p-Methoxyphenyl, o-, m- oder p-Ethoxyphenyl, o-, m- oder p-Isopropoxyphenyl, o-, m- oder p-Trifluormethylphenyl, o-, m- oder p-Fluorphenyl, o-, m- oder p-Chlorphenyl, o-, m- oder p-Bromphenyl, o-, m- oder p-Nitrophenyl.

Hal bedeutet vorzugsweise F, Cl oder Br, aber auch J.

Alkenyl hat 2-6, vorzugsweise 2, 3 oder 4 C-Atome und bedeutet bevorzugt Vinyl, Allyl, 1-Propen-1-yl, 1-Propen-2-yl, 1-, 2- oder 3-Buten-1-yl, 1-, 2- oder 3-Buten-2-yl.

Alkinyl hat 2-6, vorzugsweise 2, 3 oder 4 C-Atome und bedeutet bevorzugt Ethinyl, 1- oder 3-Propin-1-yl, 1-, 2- oder 2-Butin-1-yl.

In Ar-alkyl hat der "alkyl"-Teil 1-6, vorzugsweise 1, 2 oder 3 C-Atome. Ar-alkyl bedeutet bevorzugt Benzyl, 1- oder 2-Phenylethyl, 1-, 2- oder 3-Phenylpropyl, ferner bevorzugt o-, m- oder p-Methylbenzyl, o-, m- oder p-Methoxybenzyl, o-, m- oder
p-Trifluormethylbenzyl, o-, m- oder p-Fluorbenzyl, o-, m- oder p-Chlorbenzyl, o-, m- oder p-Brombenzyl, o-, m- oder p-Nitrobenzyl.

"Durch F substituiertes A" bedeutet vorzugsweise CH₂F, CHF₂, CF₃, CH₂-CF₃, CF₂-CF₃, CH₂-CH₂-CF₃, CH₂-CF₂-CF₃, C₃F₇, (CF₃)₂CH, iso-C₃F₇.

Cycloalkyl hat 3-8, bevorzugt 3, 4, 5 oder 6 C-Atome und bedeutet bevorzugt Cyclopropyl, Cyclobutyl, Cyclopentyl oder Cyclohexyl.

Die Reste R¹, R⁴ und R⁵ sind jeweils bevorzugt H, ferner jeweils bevorzugt A.

Der Rest R³ ist bevorzugt H, CH₂OR⁹ (insbesondere CH₂OA wie Methoxymethyl, Ethoxymethyl, Propoxymethyl, Isopropoxymethyl, Butoxymethyl, Isobutoxymethyl, Pentyloxymethyl oder Hexyloxymethyl; oder CH₂O-alkyl-Ar wie Benzyloxymethyl), CH₂R¹⁰R¹¹ (insbesondere CH₂NHR¹¹, vor allem CH₂NHA wie Methylaminomethyl, Ethylaminomethyl, Propylaminomethyl, Isopropylaminomethyl, Butylaminomethyl, Isobutylaminomethyl, Pentylaminomethyl oder Hexylaminomethyl; CH₂NH-alkylAr wie Benzylaminomethyl; oder CH₂NA-CO-NHAr wie N-Methyl-N'-Phenyl-ureidomethyl, N-Ethyl-N'-phenyl-ureidomethyl, N-Propyl-N'-phenylureidomethyl, N-Isopropyl-N'-phenyl-ureidomethyl, N-Butyl-N'-phenyl-ureidomethyl, N-Isobutyl-N'-phenyl-ureidomethyl, N-Pentyl-N'-phenyl-ureidomethyl oder N-Hexyl-N'-phenyl-ureidomethyl) oder 5-Tetrazolyl. Weiterhin ist R³ bevorzugt COOA (insbesondere Methoxycarbonyl oder Ethoxycarbonyl), CHO, CN, CH₂R⁸ (insbesondere A wie Methyl oder Ethyl) oder CONR¹⁰R¹¹ wie CONH₂.

Ganz besonders bevorzugte Reste R³ sind H oder 5-Tetrazolyl, ferner Butoxymethyl, Benzyloxymethyl, Butylaminomethyl, Benzylaminomethyl und N-Butyl-N'-phenyl-ureidomethyl.

Der Rest R⁶ ist vorzugsweise geradkettig und steht bevorzugt für A oder Alkenyl mit jeweils 3-6 C-Atomen, insbesondere Butyl, ferner Propyl, Pentyl, Hexyl, Allyl oder 1-Propenyl, ferner 1-Butenyl, 1-Pentenyl, 1-Hexenyl, 1-Propinyl, 1-Butinyl, 1-Pentinyl oder 1-Hexinyl.

Der Rest R⁷ ist vorzugsweise A, insbesondere Methyl oder Ethyl, oder CF₃.

Der Rest R⁸ ist vorzugsweise H, F, Cl, A, CN, COOH, COOA oder 5-Tetrazolyl.

Der Rest R⁹ ist vorzugsweise H, A (insbesondere mit 3-5 C-Atomen) oder Benzyl.

Der Rest R¹⁰ ist vorzugsweise H, A oder Benzyl.

Der Rest R¹¹ ist vorzugsweise H, A (insbesondere mit 1-5 C-Atomen), mehrfach durch F substituiertes Am, Benzyl, CO-A, CO-Ar, CO-CH₂C₆H₅, COOA, COOAr, COOCH₂C₆H₅, CONH₂, CONHA, CONH-Cycloalkyl, CONA₂ oder CONHAr.

Der Rest X fehlt vorzugsweise oder bedeutet vorzugsweise -NH-CO- oder -CO-NH-.

Die Verbindungen der Formel I können ein oder mehrere chirale Zentren besitzen und daher in verschiedenen - optisch-aktiven oder optisch-inaktiven - Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ig ausgedrückt werden, die der Formel I entsprechen und worin die nicht näher bezeichneten Reste die bei der Formel I angegebene Bedeutung haben, worin jedoch
- in Ia: R³ H bedeutet;
- in Ib: R³ COOH, COOA oder CONR¹⁰R¹¹ bedeutet;
- in Ic: R³ CHO oder CN bedeutet;
- In Id: R³ CH₂R⁸ bedeutet;
- in Ie: R³ CH₂OR⁸ bedeutet;
- in If: R³ NR¹⁰R¹¹ oder CH₂NR¹⁰R¹¹ bedeutet;
- in Ig: R³ 5-Tetrazolyl bedeutet.

Insbesondere sind bevorzugt Verbindungen der Formeln Ih sowie Iah bis Igh, die den Formeln I sowie Ia bis Ig entsprechen, worin jedoch zusätzlich X fehlt.

Weiterhin bevorzugt sind Verbindungen der Formeln Ii sowie Iai bis Igi, die den Formeln I sowie Ia bis Ig entsprechen, worin jedoch zusätzlich X -CO- bedeutet.

Weiterhin bevorzugt sind Verbindungen der Formeln Ij sowie Iaj bis Igj, die den Formeln I sowie Ia bis Ig entsprechen, worin jedoch zusätzlich X -O- bedeutet.

Weiterhin bevorzugt sind Verbindungen der Formeln Ik sowie Iak bis Igk, die den Formeln I sowie Ia bis Ig entsprechen, worin jedoch zusätzlich X -NH-CO- bedeutet.

Weiterhin bevorzugt sind Verbindungen der Formeln Il sowie Ial bis Igl, die den Formeln I sowie Ia bis Ig entsprechen, worin jedoch zusätzlich X -CO-NH- bedeutet.

Weiterhin bevorzugt sind Verbindungen der Formeln Im sowie Iam bis Igm, die den Formeln I sowie Ia bis Ig entsprechen, worin jedoch zusätzlich X -CH₂-O- bedeutet.

Weiterhin bevorzugt sind Verbindungen der Formeln In sowie Ian bis Ign, die den Formeln I sowie Ia bis Ig entsprechen, worin jedoch zusätzlich X -O-CH₂- bedeutet.

Vor allem bevorzugt sind Verbindungen der Formeln I sowie Ia bis In und Iah bis Ign, worin zusätzlich R¹ = H und/oder R⁴ und/oder R⁵ = H oder A und/oder R⁶ = A (insbesondere Propyl, Butyl oder Pentyl) bedeutet.

Unter diesen sind diejenigen Verbindungen bevorzugt, worin R² COOH, COOCH₃, COOC₂H₅, CN oder 5-Tetrazolyl bedeutet.

Eine ganz besonders bevorzugte Gruppe von Verbindungen entspricht der Formel I, worin
- R¹: H
- R²: COOH, COOA, CN oder 5-Tetrazolyl;
- R³: H, CH₂OA, CH₂OCH₂C₆H₅, CH₂NHA, CH₂NHCH₂C₆H₅, CH₂NACONHC₆H₅ oder 5-Tetrazolyl,
- R⁴ und R⁵: jeweils H oder A und
- R⁶: A bedeuten und
- X: fehlt.

Eine kleine ausgewählte Gruppe bevorzugter Verbindungen entspricht der Formel I, worin
- R¹, R⁴ und R⁵: jeweils H,
- R²: CN oder 5-Tetrazolyl und ,
- R³: H, CH₂OAlkyl mit 3-5 C-Atomen in der Alkylgruppe oder 5-Tetrazolyl und
- R⁶: Alkyl mit 3-5 C-Atomen bedeutet und
- X: fehlt.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; insbesondere aber US-PS 4 880 804) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können vorzugsweise erhalten werden, indem man Verbindungen der Formel II mit Verbindungen der Formel III umsetzt. Besonders die Biphenylderivate der Formel I (worin X fehlt) sind auf diesem Wege gut erhältlich.

In den Verbindungen der Formel II bedeutet E vorzugsweise Cl, Br, I oder eine reaktionsfähig funktionell abgewandelte OH-Gruppe wie Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy).

Die Umsetzung von II mit III erfolgt zweckmäßig, indem man zunächst III durch Behandeln mit einer Base in ein Salz umwandelt, z.B. mit einem Alkalimetallalkoholat wie CH₃ONa in einem Alkohol wie CH₃OH oder mit NaH oder K-tert.-Butylat in Dimethylformamid (DMF), und dieses dann in einem inerten Lösungsmittel, z.B. einem Amid wie DMF oder Dimethylacetamid oder einem Sulfoxid wie Dimethylsulfoxid (DMSO), mit II umsetzt, zweckmäßig bei Temperaturen zwischen -20 und 100°, vorzugsweise zwischen 10 und 30°.

Säureamide der Formel I (X = -NH-CO- oder -CO-NH-) sind ferner erhältlich durch Umsetzung von Verbindungen der Formel IV (oder ihrer reaktionsfähigen Derivate) mit Verbindungen der Formel V (oder ihrer reaktionsfähigen Derivate). Als reaktionsfähige Derivate der Carbonsäuren der Formeln IV und V (X¹ bzw. X² = COOH) eignen sich vorteilhaft die entsprechenden Chloride, Bromide oder Anhydride. Die Umsetzung erfolgt zweckmäßig in Gegenwart eines inerten Lösungsmittels, z.B. eines halogenierten Kohlenwasserstoffs wie Dichlormethan, Chloroform, Trichlorethen oder 1,2-Dichlorethan oder eines Ethers wie Tetrahydrofuran (THF) oder Dioxan, bei Temperaturen zwischen 0 und 150°, vorzugsweise zwischen 20 und 80°. Setzt man Säurehalogenide um, so empfiehlt sich der Zusatz einer Base, z.B. eines tertiären Amins wie Triethylamin, Pyridin oder 4-Dimethylaminopyridin.

Ether der Formel I (X = -CH₂-O- oder -O-CH₂-) sind erhältlich durch Umsetzung von Verbindungen der Formeln VI und VII (oder ihrer reaktionsfähigen Derivate). Als reaktionsfähige Derivate der Phenole VI und VII (X³ bzw. X⁴ = OH) eignen sich z.B. die entsprechenden Alkalimetall- (z.B. Na-, K-) phenolate, die auch in situ aus dem Phenol und einer Base (z.B. Kaliumcarbonat) gebildet werden können. Die Umsetzung erfolgt zweckmäßig in Gegenwart eines inerten Lösungsmittels, z.B. eines Amids wie DMF oder eines Sulfoxids wie DMSO, bei Temperaturen zwischen 0 und 150°, vorzugsweise zwischen 20 und 100°.

Die Ausgangsstoffe der Formeln II, III, IV, V, VI und VII sind teilweise bekannt. Falls sie nicht bekannt sind, können sie nach bekannten Methoden in Analogie zu bekannten Stoffen hergestellt werden. Verbindungen der Formel II sind weitgehend bekannt (vgl. z.B. EP-A2-400974). Verbindungen der Formel III sind beispielsweise erhältlich durch Umsetzung von Ketonen der Formel R⁶-CO-CH₂-R⁵ (vorzugsweise R⁶-CO-CH₃) mit Estern der Formel R⁴-COOA (vorzugsweise HCOOA, insbesondere HCOOC₂H₅) zu Dicarbonylverbindungen der Formel R⁶-CO-CR⁵=CR⁴-OH (vorzugsweise R⁶-CO-CH=CH-OH; "Enolon-form") und anschließende Kondensation mit Acetamiden der Formel R³-CH₂-CONH₂, insbesondere Cyanacetamid. So sind insbesondere 3-Cyan-4-R⁴-5-R⁵-6-R⁶-1,2-dihydro-2-oxo-pyridine (Formel III, R³ = CN, R⁴ = R⁵ = H) so erhältlich, z.B. 6-Butyl-3-cyan-1,2-dihydro-2-oxo-pyridin ("IIIb"). Aus diesen 3-Cyanverbindungen sind z.B. erhältlich: durch Hydrolyse (z.B. mit Salzsäure) die Carbonsäuren (III, R³ = COOH); daraus durch Veresterung (z.B. mit einem Alkohol A-OH in Gegenwart einer starken Säure) die Ester (III, R³ = COOA); durch Decarboxylierung die in 3-Stellung unsubstituierten 1,2-Dihydro-2-oxo-pyridine (III, R³ = H); durch Reduktion die 3-Hydroxymethylverbindungen (III, R³ = CH₂OH) oder die 3-Aminomethylverbindungen (III, R³ = CH₂NH₂) durch Oxidation der letztgenannten, z.B. mit MnO₂, die Aldehyde (III, R³ = CHO), die auch aus den Nitrilen durch Reaktion mit Diisobutylaluminiumhydrid herstellbar sind.

Partielle Hydrolyse der Nitrile (III, R³ = CN; z.B. mit 90%iger H₂SO₄; vgl. US-A1-4 137 233) führt zu den Carboxamiden (III, R³ = CONH₂), die mit NaOH/Br₂ zu den 3-Aminoverbindungen (III, R³ = NH₂) abgebaut werden können (vgl. US-A1-4 137 233).

Zur Umwandlung eines Restes R³ in Ausgangsstoffen der Formel III kann man auch die 2-Oxo-Gruppe intermediär blockieren. So kann man z.B. Nitrile (III, R³ = CN) mit Benzylchlorid/Ag₂O in siedendem Toluol in die entsprechenden 2-Benzyloxy-3-cyan-4-R⁴-5-R⁵-6-R⁶-pyridine umwandeln; durch Kochen mit ethanolischem KOH können daraus die entsprechenden 3-Carbonsäuren und durch deren Reduktion die entsprechenden 3-Hydroxymethylverbindungen erhalten werden. Alkylierung, z.B. mit Iodiden der Formel A-I, gibt die entsprechenden 2-Benzyloxy-3-alkoxymethyl-4-R⁴-5-R⁵-6-R⁶-pyridine, die zu Verbindungen der Formel III (R³ = A-O-CH₂) hydrogenolysiert werden können.

Man kann ferner eine Verbindung der Formel I aus einem ihrer funktionellen Derivate durch Behandeln mit einem solvolysierenden (z.B. hydrolysierenden) oder hydrogenolysierenden Mittel in Freiheit setzen.

So ist es möglich, nach einer der angegebenen Methoden eine Verbindung herzustellen, die der Formel I entspricht, aber an Stelle einer 5-Tetrazolylgruppe eine in 1-Stellung funktionell abgewandelte (durch eine Schutzgruppe geschützte) 5-Tetrazolylgruppe enthält. Als Schutzgruppen eignen sich beispielsweise: Triphenylmethyl, abspaltbar mit HCl in einem inerten Lösungemittel oder Lösungsmittelgemisch, z.B. Ether/Dichlormethan/Methanol; 2-Cyanethyl, abspaltbar mit NaOH in Wasser/THF; p-Nitrobenzyl, abspaltbar mit H₂/Raney-Nickel in Ethanol (vgl. EP-A2-0 291 969).

Es ist ferner möglich, eine Verbindung der Formel I in eine andere Verbindung der Formel I umzuwandeln, indem man einen oder mehrere der Reste R¹, R², R³, R⁴, R⁵ und/oder R⁶ in andere Reste R¹, R², R³, R⁴, R⁵ und/oder R⁶ umwandelt, z.B. indem man Nitrogruppen (z.B. durch Hydrierung an Raney-Nickel in einem inerten Lösungsmittel wie Methanol oder Ethanol) zu Aminogruppen reduziert und/oder freie Amino- und/oder Hydroxygruppen funktionell abwandelt und/oder funktionell abgewandelte Amino- und/oder Hydroxygruppen durch Solvolyse oder Hydrogenolyse freisetzt und/oder Halogenatome (z.B. durch Reaktion mit Alkalimetall-alkoholaten oder mit Kupfer(I)cyanid) durch OA- oder CN-Gruppen ersetzt und/oder Nitrilgruppen zu CONH₂- oder COOH-Gruppen hydrolysiert oder mit Derivaten der Stickstoffwasserstoffsäure zu Tetrazolylgruppen umsetzt und/oder CHO-Gruppen (z.B. mit NaBH₄) zur CH₂OH-Gruppen reduziert oder (z.B. mit Ammoniak oder primären Aminen in Gegenwart eines Reduktionsmittels wie NaCNBH₃) zu Aminomethyl-, A-NH-CH₂- oder Ar-alkyl-NH-CH₂-Gruppen umsetzt und/oder CONH₂-Gruppen (z.B. mit Br₂/wässeriger NaOH-Lösung) zu NH₂-Gruppen abbaut.

So kann man beispielsweise freie Hydroxy- und/oder Aminogruppen in üblicher Weise mit einem Säurechlorid oder -anhydrid acylieren oder mit einem Alkyl- oder Aralkylchlorid oder -bromid alkylieren, zweckmäßig in einem inerten Lösungsmittel wie Dichlormethan oder THF und/oder in Gegenwart einer Base wie Triethylamin oder Pyridin bei Temperaturen zwischen -60 und +30°. Eine Umwandlung primärer Aminogruppen in sekundäre oder tertiäre Aminogruppen gelingt auch durch Umsetzung mit entsprechenden Aldehyden oder Ketonen in Gegenwart eines Reduktionsmittels.

Gewünschtenfalls kann in einer Verbindung der Formel I eine funktionell abgewandelte Amino- und/oder Hydroxygruppe durch Solvolyse oder Hydrogenolyse nach üblichen Methoden in Freiheit gesetzt werden. So kann z. B. eine Verbindung der Formel I, die eine NHCOR⁷-, eine NHCOOA- oder eine AOOC-Gruppe enthält, in die entsprechende Verbindung der Formel I umgewandelt werden, die stattdessen eine NH₂- oder eine HOOC-Gruppe enthält. AOOC-Gruppen können z.B. mit NaOH oder KOH in Wasser, Wasser-THF oder Wasser-Dioxan bei Temperaturen zwischen 0 und 100° verseift werden.

Umsetzung von Nitrilen der Formel I (R² und/oder R³ = CN) mit Derivaten der Stickstoffwasserstoffsäure führt zu Tetrazolen der Formel I (R² und/oder R³ = 5-Tetrazolyl). Bevorzugt verwendet man Trialkylzinnazide wie Trimethylzinnazid in einem inerten Lösungsmittel, z.B. einem aromatischen Kohlenwasserstoff wie Toluol bei Temperaturen zwischen 20 und 150°, vorzugsweise zwischen 80 und 140°, oder Natriumazid in N-Methylpyrrolidon bei Temperaturen zwischen etwa 100 und 200°.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern, So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono-und -disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Andererseits können Verbindungen der Formel I, die COOH- oder Tetrazolylgruppen enthalten, mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden. Die Kaliumsalze der Tetrazolylderivate sind besonders bevorzugt.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vor zugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch (z.B. Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine, Diuretika, Antiphlogistika.

Die erfindungsgemäßen Substanzen werden in der Regel in Analogie zu anderen bekannten, im Handel befindlichen Präparaten, insbesondere aber in Analogie zu den in der US-PS 4 880 804 beschriebenen Verbindungen verabreicht, vorzugsweise in Dosierungen zwischen etwa 1 mg und 1 g, insbesondere zwischen 50 und 500 mg pro Dosierungseinheit. Die tägliche Dosierung liegt vorzugsweise zwischen etwa 0,1 und 50 mg/kg, insbesondere 1 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und/oder durch Kristallisation. M⁺ = Mol-Peak im Massenspektrum. Rf-Werte dünnschichtchromatographisch an Kieselgel mit Dichlormethan/Methanol (99:1, wenn nicht anders angegeben).

### Beispiel 1

Eine Lösung von 1,5 g 6-Butyl-1,2-dihydro-2-oxo-pyridin ["IIIa"; F. 69°; erhältlich durch Kondensation von 2-Hexanon mit Ethylformiat in Toluol in Gegenwart von NaOCH₃ zu 1-Hydroxy-1-hepten-3-on, Kondensation mit Cyanacetamid in Wasser in Gegenwart von Piperidin und Essigsäure bei 90° zu 6-Butyl-3-cyan-1,2-dihydro-2-oxo-pyridin ("IIIb"; F. 110°), Hydrolyse mit 37%iger Salzsäure (5 Std. Kochen) zu 6-Butyl-1,2-dihydro-2-oxo-pyridin-3-carbonsäure (F. 149°) und Decarboxylierung bei 180-200°] in 25 ml DMF wird mit 1,12 g K-tert.-Butylat versetzt. Man rührt 10 Minuten, tropft innerhalb 30 Minuten eine Lösung von 2,72 g 4'-Brommethyl-2-cyanbiphenyl ("IIa") in 15 ml DMF hinzu und rührt 16 Std. bei 20°. Man dampft ein, arbeitet wie üblich auf (pH 7; Ethylacetat), reinigt chromatographisch (Kieselgel; Dichlormethan : Methanol 98:2) und erhält 6-Butyl-1-(2'-cyan-4-biphenylylmethyl)-1,2-dihydro-2-oxo-pyridin, Rf 0,4; (M+H)⁺ 343. Als Nebenprodukt erhält man 6-Butyl-2-(2'-cyan-4-biphenylyl-methoxy)-pyridin.

Analog erhält man aus IIIa:
mit 4-Brommethyl-2'-methoxycarbonyl-benzanilid (F. 151°; erhältlich aus 4-Methyl-2'-methoxycarbonyl-benzanilid und N-Bromsuccinimid in Dichlormethan):
6-Butyl-1-[4-(2-methoxycarbonyl-anilinocarbonyl)-benzyl]-1,2-dihydro-2-oxo-pyridin;
mit 4-Brommethyl-2'-nitro-biphenyl:
6-Butyl-1-(2'-nitro-4-biphenylylmethyl)-1,2-dihydro-2-oxo-pyridin;
mit 4-Brommethyl-2'-methoxycarbonyl-benzophenon:
6-Butyl-1-[4-(2-methoxycarbonyl-benzoyl)-benzyl]-1,2-dihydro-2-oxo-pyridin;
mit 4-(2-Cyan-benzamido)-benzylbromid:
6-Butyl-1-[4-(2-cyan-benzamido)-benzyl]-1,2-dihydro-2-oxo-pyridin;
mit 4-(2-Cyan-anilinocarbonyl)-benzylbromid:
6-Butyl-1-[4-(2-cyan-anilinocarbonyl)-benzyl]-1,2-dihydro-2-oxo-pyridin.

### Beispiel 2

Analog Beispiel 1 erhält man aus IIa:
mit 6-Butyl-1,2-dihydro-2-oxo-pyridin-3-carbonsäuremethylester [F. 126°; erhältlich durch Veresterung der Carbonsäure (F. 1490; s. Beispiel 1)]:
6-Butyl-1-(2'-cyan-biphenylyl-4-methyl)-1,2-dihydro-2-oxo-pyridin-3-carbonsäuremethylester, F. 142°;
mit 6-Butyl-3-carbamoyl-1,2-dihydro-2-oxo-pyridin (F. 205°; erhältlich aus IIIb und 90%iger H₂SO₄):
6-Butyl-3-carbamoyl-1-(2'-cyan-biphenylyl-4-methyl)-1,2-dihydro-2-oxo-pyridin;
mit 6-Butyl-1,2-dihydro-2-oxo-3-formyl-pyridin (F. 118°; erhältlich aus IIIb mit Diisobutylaluminiumhydrid in Toluol bei -65°):
6-Butyl-1-(2'-cyan-biphenylyl-4-methyl)-1,2-dihydro-2-oxo-3-formyl-pyridin;
mit IIIb:
6-Butyl-1-(2'-cyan-biphenylyl-4-methyl)-1,2-dihydro-2-oxo-3-cyan-pyridin, Rf 0,25;
mit 6-Butyl-1,2-dihydro-2-oxo-3-nitro-pyridin:
6-Butyl-1-(2'-cyan-biphenylyl-4-methyl)-1,2-dihydro-2-oxo-3-nitro-pyridin;
mit 6-Butyl-1,2-dihydro-2-oxo-3-dimethylaminocarbonyl-pyridin:
6-Butyl-1-(2'-cyan-biphenylyl-4-methyl)-1,2-dihydro-2-oxo-3-dimethylaminocarbonyl-pyridin;
mit 6-Butyl-1,2-dihydro-2-oxo-3-pyrrolidino-pyridin:
6-Butyl-1-(2'-cyan-biphenylyl-4-methyl)-1,2-dihydro-2-oxo-3-pyrrolidino-pyridin;
mit 6-Butyl-1,2-dihydro-2-oxo-3-piperidino-pyridin:
6-Butyl-1-(2'-cyan-biphenylyl-4-methyl)-1,2-dihydro-2-oxo-3-piperidino-pyridin;
mit 6-Butyl-1,2-dihydro-2-oxo-3-succinimido-pyridin:
6-Butyl-1-(2'-cyan-biphenylyl-4-methyl)-1,2-dihydro-2-oxo-3-succinimido-pyridin;
mit 6-Butyl-1,2-dihydro-2-oxo-3-phthalimido-pyridin:
6-Butyl-1-(2'-cyan-biphenylyl-4-methyl)-1,2-dihydro-2-oxo-3-phthalimido-pyridin;
mit 6-Butyl-1,2-dihydro-2-oxo-3-acetamido-pyridin:
6-Butyl-1-(2'-cyan-biphenylyl-4-methyl)-1,2-dihydro-2-oxo-3-acetamido-pyridin;
mit 6-Butyl-1,2-dihydro-2-oxo-3-trimethylacetamido-pyridin:
6-Butyl-1-(2'-cyan-biphenylyl-4-methyl)-1,2-dihydro-2-oxo-3-trimethylacetamido-pyridin;
mit 6-Butyl-1,2-dihydro-2-oxo-3-benzamido-pyridin:
6-Butyl-1-(2'-cyan-biphenylyl-4-methyl)-1,2-dihydro-2-oxo-3-benzamido-pyridin;
mit 6-Butyl-1,2-dihydro-2-oxo-3-tert.-butoxycarbonylamino-pyridin:
6-Butyl-1-(2'-cyan-biphenylyl-4-methyl)-1,2-dihydro-2-oxo-3-tert.-butoxycarbonylamino-pyridin;
mit 6-Butyl-1,2-dihydro-2-oxo-3-N'-butyl-ureido-pyridin:
6-Butyl-1-(2'-cyan-biphenylyl-4-methyl)-1,2-dihydro-2-oxo-3-N'-butyl-ureido-pyridin;
mit 6-Butyl-1,2-dihydro-2-oxo-3-N'-phenyl-ureido-pyridin:
6-Butyl-1-(2'-cyan-biphenylyl-4-methyl)-1,2-dihydro-2-oxo-3-N'-phenyl-ureido-pyridin;
mit 6-Butyl-1,2-dihydro-2-oxo-3-N'-benzyl-ureido-pyridin:
6-Butyl-1-(2'-cyan-biphenylyl-4-methyl)-1,2-dihydro-2-oxo-3-N'-benzyl-ureido-pyridin;
mit 6-Butyl-1,2-dihydro-2-oxo-3-N-methyl-benzamido-pyridin:
6-Butyl-1-(2'-cyan-biphenylyl-4-methyl)-1,2-dihydro-2-oxo-3-N-methyl-benzamido-pyridin;
mit 6-Butyl-1,2-dihydro-2-oxo-3-methyl-pyridin (erhältlich durch Hydrogenolyse von 3-Acetoxymethyl-2-benzyloxy-6-butyl-pyridin an Pd-C in Ethanol bei 20° und 1 bar):
6-Butyl-1-(2'-cyan-biphenylyl-4-methyl)-1,2-dihydro-2-oxo-3-methyl-pyridin, F. 126°; Rf 0,4;
mit 6-Butyl-1,2-dihydro-2-oxo-3-fluormethyl-pyridin:
6-Butyl-1-(2'-cyan-biphenylyl-4-methyl)-1,2-dihydro-2-oxo-3-fluormethyl-pyridin;
mit 6-Butyl-1,2-dihydro-2-oxo-3-chlormethyl-pyridin:
6-Butyl-1-(2'-cyan-biphenylyl-4-methyl)-1,2-dihydro-2-oxo-3-chlormethyl-pyridin;
mit 6-Butyl-1,2-dihydro-2-oxo-3-cyanmethyl-pyridin:
6-Butyl-1-(2'-cyan-biphenylyl-4-methyl)-1,2-dihydro-2-oxo-3-cyanmethyl-pyridin;
mit 6-Butyl-1,2-dihydro-2-oxo-3-pyridin-3-essigsäureethylester:
6-Butyl-1-(2'-cyan-biphenylyl-4-methyl)-1,2-dihydro-2-oxo-pyridin-3-essigsäureethylester;
mit 6-Butyl-1,2-dihydro-2-oxo-3-methoxymethyl-pyridin:
6-Butyl-1-(2'-cyan-biphenylyl-4-methyl)-1,2-dihydro-2-oxo-3-methoxymethyl-pyridin;
mit 6-Butyl-1,2-dihydro-2-oxo-3-isopropoxymethyl-pyridin:
6-Butyl-1-(2'-cyan-biphenylyl-4-methyl)-1,2-dihydro-2-oxo-3-isopropoxymethyl-pyridin;
mit 6-Butyl-1,2-dihydro-2-oxo-3-butoxymethyl-pyridin [Öl; erhältlich durch Umsetzung von IIIb mit Benzylchlorid in Gegenwart von Ag₂O in Toluol (24 Std. Kochen) zu 2-Benzyloxy-6-butyl-3-cyan-pyridin (Öl), Hydrolyse mit wässerig-ethanolischer KOH (48 Std. Kochen) zu 2-Benzyloxy-6-butyl-nicotinsäure, Reduktion mit LiAlH₄ in THF zu 2-Benzyloxy-6-butyl-3-hydroxymethyl-pyridin, Alkylierung mit Butyl-iodid in THF in Gegenwart von NaH zu 2-Benzyloxy-6-butyl-3-butyloxymethyl-pyridin und Hydrogenolyse an 5%ig. Pd-C in Methanol]:
6-Butyl-1-(2'-cyan-biphenylyl-4-methyl)-1,2-dihydro-2-oxo-3-butoxymethyl-pyridin, Rf 0,2;
mit 6-Butyl-1,2-dihydro-2-oxo-3-benzyloxymethyl-pyridin:
6-Butyl-1-(2'-cyan-biphenylyl-4-methyl)-1,2-dihydro-2-oxo-3-benzyloxymethyl-pyridin;
mit 6-Butyl-1,2-dihydro-2-oxo-3-acetamidomethyl-pyridin:
6-Butyl-1-(2'-cyan-biphenylyl-4-methyl)-1,2-dihydro-2-oxo-3-acetamidomethyl-pyridin, F. 150°
mit 6-Butyl-1,2-dihydro-2-oxo-3-butyramidomethyl-pyridin:
6-Butyl-1-(2'-cyan-biphenylyl-4-methyl)-1,2-dihydro-2-oxo-3-butyramidomethyl-pyridin;
mit 6-Butyl-1,2-dihydro-2-oxo-3-phenylacetamidomethyl-pyridin:
6-Butyl-1-(2'-cyan-biphenylyl-4-methyl)-1,2-dihydro-2-oxo-3-phenylacetamidomethyl-pyridin;
mit 6-Butyl-1,2-dihydro-2-oxo-3-tert.-butoxycarbonylaminomethyl-pyridin:
6-Butyl-1-(2'-cyan-biphenylyl-4-methyl)-1,2-dihydro-2-oxo-3-tert.-butoxycarbonylaminomethyl-pyridin, F. 105°;
mit 6-Butyl-1,2-dihydro-2-oxo-3-phenoxycarbonylaminomethyl-pyridin:
6-Butyl-1-(2'-cyan-biphenylyl-4-methyl)-1,2-dihydro-2-oxo-3-phenoxycarbonylaminomethyl-pyridin;
mit 6-Butyl-1,2-dihydro-2-oxo-3-benzyloxycarbonylaminomethyl-pyridin:
6-Butyl-1-(2'-cyan-biphenylyl-4-methyl)-1,2-dihydro-2-oxo-3-benzyloxycarbonylaminomethyl-pyridin, Öl, Rf. 0,58 (Ethylacetat/Hexan 1:1);
mit 6-Butyl-1,2-dihydro-2-oxo-3-ureidomethyl-pyridin:
6-Butyl-1-(2'-cyan-biphenylyl-4-methyl)-1,2-dihydro-2-oxo-3-ureidomethyl-pyridin, F. 166°;
mit 6-Butyl-1,2-dihydro-2-oxo-3-(N'-butyl-ureidomethyl)-pyridin:
6-Butyl-1-(2'-cyan-biphenylyl-4-methyl)-1,2-dihydro-2-oxo-3-(N'-butyl-ureidomethyl)-pyridin, F. 61°;
mit 6-Butyl-1,2-dihydro-2-oxo-3-(N'-cyclohexyl-ureidomethyl)-pyridin:
6-Butyl-1-(2'-cyan-biphenylyl-4-methyl)-1,2-dihydro-2-oxo-3-(N'-cyclohexyl-ureidomethyl)-pyridin, F. 98°;
mit 6-Butyl-1,2-dihydro-2-oxo-3-(N'-phenyl-ureidomethyl)-pyridin:
6-Butyl-1-(2'-cyan-biphenylyl-4-methyl)-1,2-dihydro-2-oxo-3-(N'-phenyl-ureidomethyl)-pyridin, F. 89°;
mit 6-Butyl-1,2-dihydro-2-oxo-3-(N',N'-dimethyl-ureidomethyl)-pyridin:
6-Butyl-1-(2'-cyan-biphenylyl-4-methyl)-1,2-dihydro-2-oxo-3-(N',N'-dimethyl-ureidomethyl)-pyridin, F. 60°;
mit 6-Butyl-1,2-dihydro-2-oxo-3-diethylaminomethyl-pyridin:
6-Butyl-1-(2'-cyan-biphenylyl-4-methyl)-1,2-dihydro-2-oxo-3-diethylaminomethyl-pyridin;
mit 6-Butyl-1,2-dihydro-2-oxo-3-diisopropylaminomethyl-pyridin:
6-Butyl-1-(2'-cyan-biphenylyl-4-methyl)-1,2-dihydro-2-oxo-3-diisopropylaminomethyl-pyridin;
mit 6-Butyl-1,2-dihydro-2-oxo-3-pyrrolidinomethyl-pyridin:
6-Butyl-1-(2'-cyan-biphenylyl-4-methyl)-1,2-dihydro-2-oxo-3-pyrrolidinomethyl-pyridin;
mit 6-Butyl-1,2-dihydro-2-oxo-3-piperidinomethyl-pyridin:
6-Butyl-1-(2'-cyan-biphenylyl-4-methyl)-1,2-dihydro-2-oxo-3-piperidinomethyl-pyridin;
mit 6-Butyl-1,2-dihydro-2-oxo-3-(N-methyl-acetamidomethyl)-pyridin:
6-Butyl-1-(2'-cyan-biphenylyl-4-methyl)-1,2-dihydro-2-oxo-3-(N-methyl-acetamidomethyl)-pyridin;
mit 6-Butyl-1,2-dihydro-2-oxo-3-(N-isopropyl-acetamidomethyl)-pyridin:
6-Butyl-1-(2'-cyan-biphenylyl-4-methyl)-1,2-dihydro-2-oxo-3-(N-isopropyl-acetamidomethyl)-pyridin;
mit 6-Butyl-1,2-dihydro-2-oxo-3-(N-butyl-N'-methyl-ureidomethyl)-pyridin:
6-Butyl-1-(2'-cyan-biphenylyl-4-methyl)-1,2-dihydro-2-oxo-3-(N-butyl-N'-methyl-ureidomethyl)-pyridin;
mit 6-Butyl-1,2-dihydro-2-oxo-3-(N,N'-dimethyl-ureidomethyl)-pyridin:
6-Butyl-1-(2'-cyan-biphenylyl-4-methyl)-1,2-dihydro-2-oxo-3-(N,N'-dimethyl-ureidomethyl)-pyridin;
mit 6-Butyl-1,2-dihydro-2-oxo-3-(N,N',N'-trimethyl-ureidomethyl)-pyridin:
6-Butyl-1-(2'-cyan-biphenylyl-4-methyl)-1,2-dihydro-2-oxo-3-(N,N',N'-trimethyl-ureidomethyl)-pyridin;
mit 6-Butyl-1,2-dihydro-2-oxo-3-(N-butyl-N'-phenyl-ureidomethyl)-pyridin:
6-Butyl-1-(2'-cyan-biphenylyl-4-methyl)-1,2-dihydro-2-oxo-3-(N-butyl-N'-phenyl-ureidomethyl)-pyridin;
mit 6-Butyl-1,2-dihydro-2-oxo-3-(N-benzyl-N'-methyl-ureidomethyl)-pyridin:
6-Butyl-1-(2'-cyan-biphenylyl-4-methyl)-1,2-dihydro-2-oxo-3-(N-benzyl-N'-methyl-ureidomethyl)-pyridin;
mit 6-Butyl-1,2-dihydro-2-oxo-3-(N-butyl-N-isobutoxycarbonylaminomethyl)-pyridin:
6-Butyl-1-(2'-cyan-biphenylyl-4-methyl)-1,2-dihydro-2-oxo-3-(N-butyl-N-isobutoxycarbonyl-aminomethyl)-pyridin;
mit 6-Butyl-1,2-dihydro-2-oxo-3-(N-isopropyl-N-benzyloxycarbonyl-aminomethyl)-pyridin:
6-Butyl-1-(2'-cyan-biphenylyl-4-methyl)-1,2-dihydro-2-oxo-3-(N-isopropyl-N-benzyloxycarbonyl-aminomethyl)-pyridin;
mit 4-Methyl-1,2-dihydro-2-oxo-pyridin:
4-Methyl-1,2-dihydro-2-oxo-1-(2'-cyan-biphenylyl-4-methyl)-pyridin, Rf 0,1;
mit 1,2-Dihydro-2-oxo-6-phenyl-pyridin:
1-(2'-Cyan-biphenylyl-4-methyl)-1,2-dihydro-2-oxo-6-phenyl-pyridin, Rf 0,5;
mit 6-Isobutyl-1,2-dihydro-2-oxo-pyridin-3-carbonsäuremethylester (erhältlich analog IIIa aus 4-Methyl-2-pentanon über 1-Hydroxy-5-methyl-1-hexenon, 6-Isobutyl-1,2-dihydro-2-oxo-3-cyan-pyridin und 6-Isobutyl-1,2-dihydro-2-oxo-pyridin-3-carbonsäure):
1-(2'-Cyan-biphenylyl-4-methyl)-6-isobutyl-1,2-dihydro-2-oxo-pyridin-3-carbonsäuremethylester, Rf 0,5;
mit 3-Cyan-6-methyl-1,2-dihydro-2-oxo-5-propyl-pyridin (entsteht als Nebenprodukt bei der Herstellung von IIIb):
3-Cyan-1-(2'-cyan-biphenylyl-4-methyl)-6-methyl-1,2-dihydro-2-oxo-5-propyl-pyridin, F. 186°;
mit 6-Methyl-, 6-Ethyl-, 6-Propyl- oder 6-Pentyl-1,2-dihydro-2-oxo-pyridin:
1-(2'-Cyan-biphenylyl-4-methyl)-6-methyl-1,2-dihydro-2-oxo-pyridin
1-(2'-Cyan-biphenylyl-4-methyl)-6-ethyl-1,2-dihydro-2-oxo-pyridin
1-(2'Cyan-biphenylyl-4-methyl)-1,2-dihydro-2-oxo-6-propyl-1,2-dihydro-2-oxo-pyridin
1-(2'-Cyan-biphenylyl-4-methyl)-1,2-dihydro-2-oxo-6-pentyl-1,2-dihydro-2-oxo-pyridin.

### Beispiel 3

a) Eine Lösung von 2,8 g 3-tert.-Butoxycarbonylaminomethyl-6-butyl-1,2-dihydro-2-oxopyridin [F. 147°; erhältlich durch Hydrierung von 6-Butyl-3-cyan-1,2-dihydro-2-oxopyridin anRaney-Ni in Ethanol in Gegenwart von NH₃ bei 5 bar zu 3-Aminomethyl-6-butyl-1,2-dihydro-2-oxo-pyridin (F. 83°) und Reaktion mit 2,2,8,8-Tetramethyl-3,5,7-trioxa-4,6-dioxononan in THF] in 35 ml DMF wird unter Rühren mit 1,1 g Kalium-tert.-butylat, dann nach 1/2 Std. mit 5,45 g 4'-Brommethyl-2-[1(oder 2)-triphenylmethyl-1H(oder 2H)-5-tetrazolyl]-biphenyl (vgl. EP-A2-0 392 317; dort als "1-triphenylmethyl-1H" bezeichnet, Struktur jedoch nicht bewiesen) versetzt und 3 Std. bei 20° gerührt. Nach üblicher Aufarbeitung (Ethylacetat) erhält man 3-tert.-Butoxycarbonylaminomethyl-6-butyl-1,2-dihydro-2-oxo-1-[2'-(1-triphenylmethyl-1H-5-tetrazolyl)-biphenylyl-4-methyl]-pyridin.
b) Eine Lösung von 7,45 g des nach a) erhaltenen Produkts in 20 ml Dichlormethan und 20 ml Methanol wird mit 20 ml etherischer Salzsäure versetzt und 3 Std. bei 20° gerührt. Man dampft ein, arbeitet wie üblich auf und erhält nach chromatographischer Abtrennung des gebildeten Triphenylcarbinols 3-tert.-Butoxycarbonylaminomethyl-6-butyl-1,2-dihydro-2-oxo-1-[2'-(5-tetrazolyl)-biphenylyl-4-methyl]-pyridin, F. 112°.

Analog erhält man mit in Beispiel 2 angegebenen 6-Butyl-1,2-dihydro-2-oxo-pyridinen über die entsprechenden 6-Butyl-1,2-dihydro-2-oxo-1-[2'-(1(oder 2)-triphenylmethyl-1H(oder 2H)-5-tetrazolyl)-biphenylyl-4-methyl]-pyridine die nachstehenden 6-Butyl-1,2-dihydro-2-oxo-1-[2'-(5-tetrazolyl)-biphenylyl-4-methyl]-pyridine:
3-Pyrrolidino-
3-Piperidino-
3-Succinimido-
3-Phthalimido-
3-Benzamido-
3-tert.-Butoxycarbonylamino-
3-N'-Butyl-ureido-
3-N'-Phenyl-ureido-
3-N'-Benzyl-ureido-
3-N-Methyl-benzamido-
3-Cyanmethyl-
3-Ethoxycarbonylmethyl-
3-(5-Tetrazolyl-methyl)-
3-Acetamidomethyl-, F. 183° (Zers.)
3-Butyramidomethyl-
3-Phenylacetamidomethyl-
3-tert.-Butoxycarbonylaminomethyl-, F. 112°
3-Phenoxycarbonylaminomethyl-
3-Benzyloxycarbonylaminomethyl-
3-Ureidomethyl-
3-N'-Butyl-ureidomethyl-, F. 275°
3-N'-Cyclohexyl-ureidomethyl-, 298°
3-N'-Phenyl-ureidomethyl-, F. 297°
3-N',N'-Dimethyl-ureidomethyl-
3-Diethylaminomethyl-
3-Diisopropylaminomethyl-
3-Pyrrolidinomethyl-
3-Piperidinomethyl-
3-(N-Methyl-acetamidomethyl)-
3-(N-Isopropyl-acetamidomethyl)-
3-(N-Butyl-N'-methyl-ureidomethyl)-
3-(N,N'-Dimethyl-ureidomethyl)-
3-(N,N',N'-Trimethyl-ureidomethyl)-
3-(N-Butyl-N'-phenyl-ureidomethyl)-
3-(N-Benzyl-N'-methyl-ureidomethyl)-
3-(N-Butyl-N-isobutoxycarbonyl-aminomethyl)-
3-(N-Isopropyl-N-benzyloxycarbonyl-aminomethyl)-.

Analog erhält man aus 6-Butyl-1-(2'-cyan-biphenylyl-4-methyl)-1,2-dihydro-2-oxo-3-(1(oder 2)-triphenylmethyl -1H(oder 2H)-5-tetrazolyl-methyl)-pyridin das 6-Butyl-1-(2'-cyan-biphenylyl-4-methyl)-1,2-dihydro-2-oxo-3-(1H-5-tetrazolylmethyl)-pyridin.

### Beispiel 4

Ein Gemisch von 1 g 1-p-Aminobenzyl-6-butyl-1,2-dihydro-2-oxo-pyridin, 0,6 g Phthalsäureanhydrid und 40 ml CHCl₃ wird 16 Std. bei 20° gerührt. Das ausgefallene Phthalsäuremono-4-(6-butyl-1,2-dihydro-2-oxo-1-pyridyl-methyl)-anilid wird abfiltriert.

### Herstellung des Ausgangsmaterials:

(a) Man löst 3 g IIIa in 75 ml Methanol und tropft unter Rühren bei 20° eine Lösung von 0,4 g Na in 10 ml Methanol hinzu. Man rührt noch 45 Min., dampft ein, löst den Rückstand in 30 ml DMF, kühlt auf 0°, gibt bei diesr Temperatur eine Lösung von 3,7 g p-Nitrobenzylbromid hinzu und rührt 16 Std. bei 20°. Man dampft ein, arbeitet wie üblich auf und erhält 6-Butyl-1-p-nitrobenzyl-1,2-dihydro-2-oxo-pyridin.
(b) Eine Lösung von 1,7 g 6-Butyl-1-p-nitrobenzyl-1,2-dihydro-2-oxo-pyridin in 50 ml Methanol wird an 1,7 g Raney-Ni bis zum Stillstand der H₂-Aufnahme bei 20° hydriert. Man filtriert, dampft ein und erhält 1-p-Aminobenzyl-6-butyl-1,2-dihydro-2-oxo-pyridin.

### Beispiel 5

Ein Gemisch von 2,56 g 6-Butyl-1-p-aminobenzyl-1,2-dihydro-2-oxo-pyridin, 3 ml Triethylamin, 0,5 g 4-Dimethylamino-pyridin und 120 ml CH₂Cl₂ wird auf 5° gekühlt und tropfenweise mit einer Lösung von 2,88 g o-Trifluormethansulfonamidobenzoylchlorid in 20 ml CH₂Cl₂ versetzt. Man rührt noch 16 Std. bei 20°, dampft ein, arbeitet wie üblich auf und erhält 6-Butyl-1-[4-(o-trifluormethansulfonamido-benzamido)-benzyl]-1,2-dihydro-2-oxo-pyridin.

### Beispiel 6

Ein Gemisch von 2,85 g 6-Butyl-1-p-carboxybenzyl-1,2-dihydro-2-oxo-pyridin, 12 g Thionylchlorid und 35 ml CHCl₃ wird 6 Std. gekocht und eingedampft. Das erhaltene rohe Säurechlorid wird durch mehrfaches Lösen in Toluol und Eindampfen von Thionylchlorid-Resten befreit und in 50 ml THF gelöst. Man tropft diese Lösung zu einer Lösung von 1,7 g Anthranilsäure und 0,8 g NaOH in 100 ml Wasser, rührt 24 Std. und säuert mit Salzsäure bis pH 5 an. Nach üblicher Aufarbeitung erhält man 6-Butyl-1-[4-(2-carboxy-anilinocarbonyl)-benzyl]-1,2-dihydro-2-oxo-pyridin.

### Herstellung der Ausgangsstoffe:

(a) Analog Beispiel 1 setzt man IIIa mit p-Brommethylbenzonitril um und erhält nach Chromatographie an Kieselgel (Methyl-tert.-butylether/Methanol) 6-Butyl-1-p-cyanbenzyl-1,2-dihydro-2-oxo-pyridin.
(b) Man kocht ein Gemisch von 1 g 6-Butyl-1-p-cyanbenzyl-1,2-dihydro-2-oxo-pyridin, 0,7 g KOH, 20 ml Ethanol und 5 ml Wasser 24 Std. unter Rühren, dampft ein, löst in Wasser und säuert mit Salzsäure an. Das ausgefallene 6-Butyl-1-p-carboxybenzyl-1,2-dihydro-2-oxo-pyridin wird abfiltriert und mit Wasser gewaschen.

### Beispiel 7

Ein Gemisch von 1,19 g o-Cyanphenol, 0,75 g K₂CO₃ und 10 ml DMF wird 0,5 Std. gerührt. Man tropft eine Lösung von 3,34 g 1-p-Brommethyl-benzyl-6-butyl-1,2-dihydro-2-oxo-pyridin (erhältlich durch Reaktion von IIIa mit p-Benzyloxymethylbenzylbromid zu 1-Benzyloxymethyl-benzyl-6-butyl-1,2-dihydro-2-oxo-pyridin, Hydrogenolyse zur 3-p-Hydroxymethylbenzyl-Verbindung und Umsetzung mit PBr₃) in 20 ml DMF hinzu, erhitzt 8 Std. auf 90°, dampft ein und erhält nach üblicher Aufarbeitung 6-Butyl-1-(4-o-cyan-phenoxymethylbenzyl)-1,2-dihydro-2-oxo-pyridin.

### Beispiel 8

Ein Gemisch von 2,57 6-Butyl-1-p-hydroxybenzyl-1,2-dihydro-2 oxo-pyridin (erhältlich aus IIIa und p-Hydroxybenzylbromid), 0,5 g CH₃ONa und und 40 ml DMSO wird 0,5 Std. gerührt. Man tropft eine Lösung von 2,2 g o-Cyanbenzylbromid in 15 ml DMSO hinzu, rührt 16 Std. bei 20°, dampft ein und erhält nach üblicher Aufarbeitung 6-Butyl-1-(4-o-cyan-benzyloxybenzyl)-1,2-dihydro-2-oxo-pyridin.

### Beispiel 9

Ein Gemisch von 404 mg 6-Butyl-1-[4-(2-methoxycarbonyl-anilino-carbonyl)-benzyl]-1,2-dihydro-2-oxo-pyridin, 10 ml 0,1 n wässerige NaOH-Lösung und 17 ml THF wird 48 Std. bei 20° stehengelassen. Man dampft das THF ab, säuert mit HCl an, extrahiert mit Dichlormethan, trocknet über Na₂SO₄ und erhält nach dem Eindampfen 6-Butyl-1-[4-(2-carboxy-anilino-carbonyl)-benzyl]-1,2-dihydro-2-oxo-pyridin.

Analog erhält man aus den entsprechenden Methyl- oder Ethylestern:
6-Butyl-1,2-dihydro-2-oxo-1-(2'-cyan-biphenylyl-4-methyl)-pyridin-3-carbonsäure
6-Butyl-1,2-dihydro-2-oxo-1-(2'-cyan-biphenylyl-4-methyl)-pyridin-3-essigsäure
6-Butyl-1-[2'-(5-tetrazolyl)-biphenylyl-4-methyl]-1,2-dihydro-2-oxopyridin-3-essigsäure.

### Beispiel 10

Ein Gemisch von 400 mg 6-Butyl-3-cyanmethyl-1-(2'-methoxycarbonyl-biphenyly-4-methyl)-1,2-dihydro-2-oxo-pyridin und 2,2 ml 1 n wässeriger KOH-Lösung wird 3 Std. gekocht, abgekühlt und mit Salzsäure angesäuert. Nach üblicher Aufarbeitung erhält man 6-Butyl-3-carbamoylmethyl-1-(2'-carboxybiphenylyl-4-methyl)-1,2-dihydro-2-oxo-pyridin.

### Beispiel 11

Ein Gemisch von 472 mg 3-tert.-Butoxycarbonylaminomethyl-6-butyl-1-(2'-cyan-biphenylyl-4-methyl)-1,2-dihydro-2-oxo-pyridin, 206 mg Trimethylzinnazid und 12 ml Xylol wird 96 Std. gekocht; nach 48 Std. gibt man nochmals 0,2 g Azid hinzu. Man kühlt ab, versetzt mit etherischer Salzsäure und dampft ein.

Chromatograhie des Rückstandes (Kieselgel; Dichlormethan/Methanol 95:5) liefert 3-tert.-Butoxycarbonylaminomethyl-6-butyl-1,2-dihydro-2-oxo-1-[2'-(5-tetrazolyl)-biphenylyl-4-methyl]-pyridin, F. 112°. In üblicher Weise wird daraus das entsprechende K-Salz hergestellt.

### Beispiel 12

Analog Beispiel 11, aber unter Verwendung von 2 Äquivalenten Trimethylzinnazid, erhält man aus 6-Butyl-3-cyan-1-(2'-cyan-biphenylyl-4-methyl)-1,2-dihydro-2-oxo-pyridin das 6-Butyl-1,2-dihydro-2-oxo-3-(5-tetrazolyl)-1-[2'-(5-tetrazolyl)-biphenylyl-4-methyl]-pyridin, F. 259° (Zers.); Rf 0,1 (7:3). Di-K-Salz, F. 235°. Daneben erhält man 6-Butyl-3-cyan-1,2-dihydro-2-oxo-1-[2'-(5-tetrazolyl)-biphenylyl-4-methyl]-pyridin (chromatographisch abtrennbar).

Analog kann man aus 3-Cyan-1-(2'-cyan-biphenylyl-4-methyl)-6-methyl-1,2-dihydro-2-oxo-5-propyl-pyridin das 6-Methyl-1,2-dihydro-2-oxo-5-propyl-3-(5-tetrazolyl)-1-[2'-(5-tetrazolyl)-biphenylyl-4-methyl]-pyridin herstellen.

### Beispiel 13

Ein Gemisch von 472 mg 3-tert.-Butoxycarbonylaminomethyl-6-butyl-1-(2'-cyan-biphenylyl-4-methyl)-1,2-dihydro-2-oxo-pyridin, 700 mg NH₄Cl, 700 mg NaN₃ und 4 ml DMF wird 36 Std. bei 120° gerührt. Man kühlt ab, filtriert das gebildete NaCl ab, dampft ein, arbeitet mit wässeriger Salzsäure/Dichlormethan wie üblich auf und erhält 3-tert.-Butoxycarbonylaminomethyl-6-butyl-1,2-dihydro-2-oxo-1-[2'-(5-tetrazolyl)-biphenylyl-4-methyl]-pyridin, F. 112°.

### Beispiel 14

a) Eine Lösung von 1 g 6-Butyl-1-(2'-nitro-biphenylyl-4-methyl)-1,2-dihydro-2-oxopyridin in 30 ml Ethanol wird an 1 g Raney-Ni bis zum Stillstand der H₂-Aufnahme bei 20° hydriert. Man filtriert, dampft ein und erhält 1-(2'-Amino-biphenylyl-4-methyl)-6-butyl-1,2-dihydro-2-oxo-pyridin.
b) Eine Lösung von 2,82 g Trifluormethansulfonsäureanhydrid in 10 ml CH₂Cl₂ wird zugetropft zu einer Lösung von 3,32 g 1-(2'-Amino-biphenylyl-4-methyl)-6-butyl-1,2-dihydro-2-oxo-pyridin und 1,01 g Triethylamin in 30 ml CH₂Cl₂ bei -50 bis -60°. Man läßt das Gemisch sich auf 20° erwärmen, gießt in verdünnte Essigsäure und erhält nach üblicher Aufarbeitung 6-Butyl-1-(2'-trifluormethansulfonamidobiphenylyl-4-methyl)-1,2-dihydro-2-oxo-pyridin.

### Beispiel 15

Eine Lösung von 370 mg 6-Butyl-1-(2'-cyan-biphenylyl-4-methyl)-3-formyl-1,2-dihydro-2-oxo-pyridin in 5 ml Isopropanol wird unter Rühren bei 20° mit 15 mg NaBH₄ versetzt. Nach weiterem Rühren (1 Std.) tropft man verdünnte Salzsäure hinzu, bis die H₂-Entwicklung aufgehört hat, und dampft ein. Nach üblicher Aufarbeitung erhält man 6-Butyl-1-(2'-cyan-biphenylyl-4-methyl)-3-hydroxymethyl-1,2-dihydro-2-oxo-pyridin.

### Beispiel 16

Zu einer Lösung von 280 mg NaOH in 6 ml Wasser tropft man unter Rühren bei 0° 0,07 ml Brom. Dann werden 360 mg 6-Butyl-3-carbamoyl-1-(2'-cyan-biphenylyl-4-methyl)-1,2-dihydro-2-oxo-pyridin hinzugefügt. Man erhitzt 3 Std. auf dem Dampfbad, säuert mit 6 n Salzsäure an und rührt weitere 30 Minuten. Es wird mit 10%iger KHCO₃-Lösung neutralisiert, abgekühlt und wie üblich aufgearbeitet. Man erhält 3-Amino-6-Butyl-1-(2'-cyan-biphenylyl-4-methyl)-1,2-dihydro-2-oxo-pyridin.

### Beispiel 17

Eine Lösung von 1 g 3-tert.-Butoxycarbonylaminomethyl-6-butyl-1-(2'-cyan-biphenylyl-4-methyl)-1,2-dihydro-2-oxo-pyridin in 20 ml 4 n HCl-Lösung in Dioxan wird 1 Std. bei 20° gerührt und dann eingedampft. Man erhält 3-Aminomethyl-6-butyl-1-(2'-cyan-biphenylyl-4-methyl)-1,2-dihydro-2-oxo-pyridin in Form des Hydrochlorids; freie Base, F. 118°.

### Beispiel 18

Eine Suspension von 370 mg 6-Butyl-1-(2'-cyan-biphenylyl-4-methyl)-3-formyl-1,2-dihydro-2-oxo-pyridin, 770 mg Ammoniumacetat, 61 mg NaBH₃CN und 400 mg gepulvertem Molekularsieb 3Å in 10 ml Isopropanol wird unter Argon 3 Tage bei 20° gerührt. Man filtriert, wäscht mit Methanol und dampft ein. Nach üblicher Aufarbeitung (Natronlauge/Dichlormethan) erhält man 3-Aminomethyl-6-butyl-1-(2'-cyan-biphenylyl-4-methyl)-1,2-dihydro-2-oxopyridin, F. 118°.

Analog erhält man aus 6-Butyl-3-formyl-1,2-dihydro-2-oxo⁻1-[2'-(5-tetrazolyl)-biphenylyl-4-methyl]-pyridin das 3-Aminomethyl-6-butyl-1,2-dihydro-2-oxo-1-[2'(5-tetrazolyl)biphenylyl-4-methyl]-pyridin, Zersetzung ab 75°.

### Beispiel 19

Ein Gemisch von 3,7 g 6-Butyl-1-(2'-cyan-biphenylyl-4-methyl)-3-formyl-1,2-dihydro-2-oxo-pyridin, 0,59 g Isopropylamin und 3,55 g Titantetraisopropoxid wird 2 Std. bei 20° unter N₂ gerührt. Man gibt 10 ml absolutes Ethanol, dann 610 mg NaBH₃CN hinzu, rührt noch 20 Std., fügt dann 2 ml Wasser hinzu und filtriert ab. Das Filtrat wird eingedampft. Nach üblicher Aufarbeitung erhält man 6-Butyl-1-(2'-cyan-biphenylyl-4-methyl)-3-isopropylaminomethyl-1,2-dihydro-2-oxo-pyridin.

Analog erhält man mit Butylamin oder Benzylamin:
6-Butyl-3-butylaminomethyl-1-(2'-cyan-biphenylyl-4-methyl)-1,2-dihydro-2-oxo-pyridin
3-Benzylaminomethyl-6-butyl-1-(2'-cyan-biphenylyl-4-methyl)-1,2-dihydro-2-oxo-pyridin, Öl, Rf. 0,15 (Dichlormethan/Methanol 95:5)
sowie aus 6-Butyl-3-formyl-1-[2'-(5-tetrazolyl)-biphenylyl-4-methyl]-1,2-dihydro-2-oxo-pyridin:
6-Butyl-3-isopropylaminomethyl-1-[2'-(5-tetrazolyl)-biphenylyl-4-methyl]-1,2-dihydro-2-oxo-pyridin
6-Butyl-3-butylaminomethyl-1-[2'-(5-tetrazolyl)-biphenylyl-4-methyl]-1,2-dihydro-2-oxo-pyridin
3-Benzylaminomethyl-6-butyl-1-[2'-(5-tetrazolyl)-biphenylyl-4-methyl]-1,2-dihydro-2-oxo-pyridin.

### Beispiel 20

Analog Beispiel 19 erhält man aus 3-Amino-6-butyl-1-(2'-cyan-bihenylyl-4-methyl)-1,2-dihydro-2-oxopyridin und Aceton/Titantetraisopropoxid/NaBH₃CN das 6-Butyl-1-(2'-cyan-biphenylyl-4-methyl)-3-isopropylamino-1,2-dihydro-2-oxo-pyridin.

Analog erhält man mit Butyraldehyd, Benzaldehyd oder Hexafluoraceton aus den entsprechenden 3-Amino- oder 3-Aminomethyl-Verbindungen:
6-Butyl-3-butylamino-1-(2'-cyan-biphenylyl-4-methyl)-1,2-dihydro-2-oxo-pyridin
3-Benzylamino-6-butyl-1-(2'-cyan-biphenylyl-4-methyl)-1,2-dihydro-2-oxo-pyridin
6-Butyl-3-(1,1,1,3,3,3-hexafluor-2-propylamino)-1-(2'-cyan-biphenylyl-4-methyl)-1,2-dihydro-2-oxo-pyridin
6-Butyl-3-(1,1,1,3,3,3-hexafluor-2-propylaminomethyl)-1-(2'-cyan-biphenylyl-4-methyl)-1,2-dihydro-2-oxo-pyridin
3-Benzylamino-6-butyl-1,2-dihydro-2-oxo-1-[2'-(5-tetrazolyl)-biphenylyl-4-methyl]-pyridin
6-Butyl-3-(1,1,1,3,3,3-hexafluor-2-propylamino)-1,2-dihydro-2-oxo-1-[2'-(5-tetrazolyl)-biphenylyl-4-methyl]-pyridin
6-Butyl-3-(1,1,1,3,3,3-hexafluor-2-propylamino-methyl)-1,2-dihydro-2-oxo-1-[2'-(5-tetrazolyl)-biphenylyl-4-methyl]-pyridin.

### Beispiel 21

Analog Beispiel 1 erhält man aus IIa mit 6-Butyl-1,2-dihydro-3-methylsulfonylaminomethyl-, -3-trifluormethylsulfonylaminomethyl-, -3-phenylsulfonylaminomethyl- oder -3-p-tolylsulfonylaminomethyl-2-oxo-pyridin die nachstehenden 6-Butyl-1-(2'-cyanbiphenylal-4-methyl)-1,2-dihydro-2-oxo-pyridine:
3-Methylsulfonylaminomethyl-, F. 59°
3-Trifluormethylsulfonylaminomethyl-
3-Phenylsulfonylaminomethyl-
3-p-Tolylsulfonylaminomethyl-, F. 119°.

### Beispiel 22

Analog Beispiel 11 erhält man aus den in Beispiel 21 genannten 2'-Cyanbiphenylylverbindungen mit Trimethylzinnazid die nachstehenden 6-Butyl-1-[2'-(5-tetrazolyl)-biphenylyl-4-methyl]-1,2-dihydro-2-oxo-pyridine:
3-Methylsulfonylaminomethyl-
3-Trifluormethylsulfonylaminomethyl-
3-Phenylsulfonylaminomethyl-
3-p-Tolylsulfonylaminomethyl-.

Die nachstehenden Beispiele betreffen pharmazeutische Zubereitungen, die Wirkstoffe der Formel I oder ihre Salze enthalten.

### Beispiel A: Tabletten und Dragees

In üblicher Weise werden Tabletten folgender Zusammensetzung gepreßt, die bei Bedarf mit einer üblichen Drageedecke auf Sucrosegrundlage überzogen werden:

| | |
|---|---|
| Wirkstoff der Formel I | 100 mg |
| Mikrokristalline Cellulose | 278,8 mg |
| Lactose | 110 mg |
| Maisstärke | 11 mg |
| Magnesiumstearat | 5 mg |
| Feinteiliges Siliciumdioxid | 0,2 mg |

### Beispiel B: Hartgelatine-Kapseln

Übliche zweiteilige Hartgelatine-Kapseln werden jeweils gefüllt mit

| | |
|---|---|
| Wirkstoff der Formel I | 100 mg |
| Lactose | 150 mg |
| Cellulose | 50 mg |
| Magnesiumstearat | 6 mg |

### Beispiel C: Weichgelatine-Kapseln

Übliche Weichgelatine-Kapseln werden mit einem Gemisch aus jeweils 50 mg Wirkstoff der Formel I und 250 mg Olivenöl gefüllt.

### Beispiel D: Ampullen

Eine Lösung von 200 g Wirkstoff der Formel I in 2 kg 1,2-Propandiol wird mit Wasser auf 10 l aufgefüllt und in Ampullen gefüllt, so daß jede Ampulle 20 mg Wirkstoff enthält.

## Patentansprüche

1. 1,2-Dihydro-2-oxopyridine der Formel I 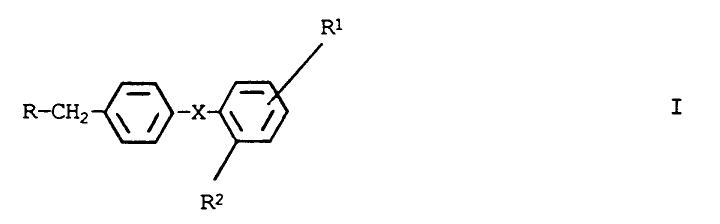 worin
R den Rest 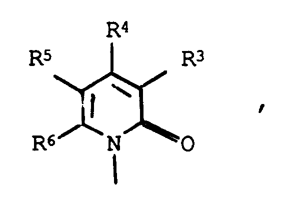
R¹ H, Hal, A, OA oder NO₂,
R² COOH, COOA, CN, NO₂, NH₂, NHCOR⁷, NHSO₂R⁷ oder 5-Tetrazolyl,
R³ H, COOH, COOA, CHO, CN, NO₂, CH₂R⁸, CH₂OR⁹, NR¹⁰R¹¹, CH₂NR¹⁰R¹¹, CONR¹⁰R¹¹ oder 5-Tetrazolyl,
R⁴ H oder A,
R⁵, R⁶ und R¹⁰ jeweils H, A, Alkenyl mit 2-6 C-Atomen, Alkinyl mit 2-6 C-Atomen, Ar oder Ar-alkyl mit 1-6 C-Atomen im "alkyl"-Teil,
R⁷ A oder ein- oder mehrfach durch F substituiertes A,
R⁸ H, Hal, A, Ar, CN, COOH, COOA, CH₂COOH, CH₂COOA oder 5-Tetrazolyl,
R⁹ H, A, Ar, Ar-alkyl mit 1-6 C-Atomen im "alkyl"-Teil, COA, COAr, COOA, COOAr, CONR¹²R¹³, COO-alkyl-Ar oder A-O-alkyl mit jeweils 1-6 C-Atomen im "alkyl"-Teil,
R¹¹ H, A, ein- oder mehrfach durch F substituiertes A, Ar, Ar-alkyl mit 1-6 C-Atomen im "alkyl"-Teil, CO-A, CO-Ar, CO-alkyl-Ar mit 1-6 C-Atomen im "alkyl"-Teil, COOA, COOAr, COO-alkyl-Ar mit 1-6 C-Atomen im "alkyl"-Teil, CONR¹²R¹³, SO₂R⁷ oder SO₂-Ar,
NR¹⁰R¹¹ auch Pyrrolidino, Piperidino, Morpholino, Succinimido oder Phthalimido,
R¹² und R¹³ jeweils H, A, Cycloalkyl mit 3-8 C-Atomen, Alkenyl mit 2-6 C-Atomen, Alkinyl mit 2-6 C-Atomen oder Ar,
X fehlt, -CO-, -O-, -NH-CO-, -CO-NH-, -CH₂-O-oder -O-CH₂-,
A Alkyl mit 1-6 C-Atomen,
Ar unsubstituiertes oder einfach durch A, OA, CF₃, Hal oder NO₂ substituiertes Phenyl und
Hal F, Cl, Br oder I bedeuten,
worin jedoch mindestens eine der folgenden Bedingungen erfüllt sein muß:
(a) R¹ bedeutet Hal, A, OA oder NO₂,
(b) R² bedeutet CN, NO₂, NH₂, NHCOR⁷ oder NHSO₂R⁷,
(c) R³ bedeutet CH₂Ar, CH₂CN, CH₂COOH, CH₂COOA, CH₂CH₂COOH, CH₂CH₂COOA, CH₂-(5-Tetrazolyl); CH₂OAr, CH₂O-alkyl-Ar' (worin Ar' eine einfach durch A, OA, CF₃, Hal oder NO₂ substituierte Phenylgruppe bedeutet), CH₂O-CO-Ar', CH₂OCOOAr, CH₂OCONR¹⁴R¹⁵ (worin R¹⁴ und R¹⁵ jeweils unabhängig voneinander Cycloalkyl, Alkenyl, Alkinyl oder Ar bedeuten, einer der Reste R¹⁴ und R¹⁵ auch H oder A bedeuten kann), CH₂O-COO-alkyl-Ar, CH₂O-alkyl-O-A; NR¹⁶R¹⁷ (worin R¹⁶ Alkenyl, Alkinyl, Ar oder Ar-alkyl, R¹⁷ ein- oder mehrfach durch F substituiertes A, Ar, Ar-alkyl, CO-Ar, CO-alkyl-Ar, COOA, COOAr, COO-alkyl-Ar oder CONR¹²R¹³ bedeuten, einer der Reste R¹⁶ und R¹⁷ auch H oder A, die Gruppe NR¹⁶R¹⁷ auch Pyrrolidino, Piperidino, Morpholino, Succinimido oder Phthalimido bedeuten kann); CH₂NR¹⁰R¹¹; CONR¹⁸R¹⁹ (worin R¹⁸ Alkenyl, Alkinyl, Ar oder Aralkyl, R¹⁹ ein- oder mehrfach durch F substituiertes A, Ar', Ar-alkyl, CO-Ar, CO-alkyl-Ar, COOA, COOAr, COO-alkyl-Ar oder CONR¹²R¹³ bedeuten, einer der Reste R¹⁸ und R¹⁹ auch H oder A, die Gruppe NR¹⁸R¹⁹ auch Succinimido oder Phthalimido bedeuten kann);
(d) R⁵ bedeutet Alkinyl, Ar' oder Ar-alkyl,
(e) R⁶ bedeutet Alkinyl, Ar' oder Ar-alkyl,
(f) X bedeutet -CO-, -O-, -NH-CO-, -CO-NH-, -CH₂-O- oder -O-CH₂-,
sowie ihre Salze.

2. a) 6-Butyl-1,2-dihydro-2-oxo-1-[2'-(5-tetrazolyl)-biphenylyl-4-methyl]-pyridin.
b) 6-Butyl-1,2-dihydro-2-oxo-3-(5-tetrazolyl)-1-[2'-(5-tetrazolyl)-biphenylyl-4-methyl]-pyridin.

3. Verfahren zur Herstellung von 1,2-Dihydro-2-oxo-pyridinen der Formel I nach Anspruch 1 sowie ihrer Salze, dadurch gekennzeichnet, daß man
(a) eine Verbindung der Formel II 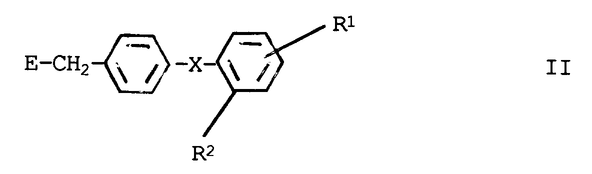 worin
E Cl, Br, I oder eine freie oder reaktionsfähig funktionell abgewandelte OH-Gruppe bedeutet und
R¹, R² und X die in Anspruch 1 angegebene Bedeutung haben,
mit einer Verbindung der Formel III
H-R III
worin
R die in Anspruch 1 angegebene Bedeutung hat,
oder
(b) zur Herstellung einer Verbindung der Formel I, worin X -NH-CO- oder -CO-NH- bedeutet, eine Verbindung der Formel IV 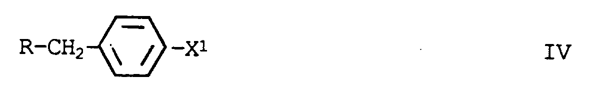 worin
X¹ NH₂ oder COOH bedeutet und
R die in Anspruch 1 angegebene Bedeutung hat
oder ein reaktionsfähiges Derivat dieser Verbindung mit einer Verbindung der Formel V 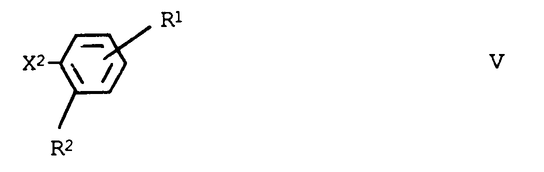 worin
X² COOH (falls X¹ NH₂ ist) oder NH₂ (falls X¹ COOH ist) bedeutet und
R¹ und R² die in Anspruch 1 angegebenen Bedeutungen haben
oder mit einem reaktionsfähigen Derivat dieser Verbindung oder
(c) zur Herstellung einer Verbindung der Formel I, worin X -CH₂-O- oder -O-CH₂- bedeutet, eine Verbindung der Formel VI 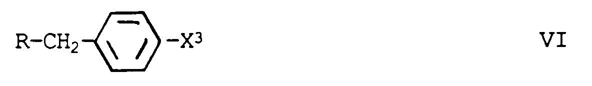 worin
X³ CH₂E oder OH bedeutet und
R die in Anspruch 1 angegebene Bedeutung hat
oder ein reaktionsfähiges Derivat dieser Verbindung mit einer Verbindung der Formel VII 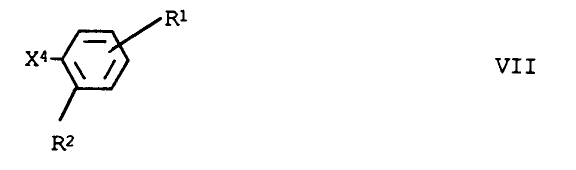 worin
X⁴ OH (falls X³ CH₂E ist) oder CH₂E (falls X³ OH ist) bedeutet und
R¹ und R² die in Anspruch 1 angegebenen Bedeutungen haben
oder mit einem reaktionsfähigen Derivat dieser Verbindung
umsetzt,
oder daß man eine Verbindung der Formel I aus einem ihrer funktionellen Derivate durch Behandeln mit einem solvolysierenden oder hydrogenolysierenden Mittel in Freiheit setzt,
und/oder daß man in einer Verbindung der Formel I einen oder mehrere Rest(e) R¹, R², R³, R⁴, R⁵ und/oder R⁶ in einen oder mehrere andere Reste R¹, R², R³, R⁴, R⁵ und/oder R⁶ umwandelt und/oder eine Base oder Säure der Formel I in eines ihrer Salze umwandelt.

4. Verfahren zur Herstellung pharmazeutischer Zubereitungen, dadurch gekennzeichnet, daß man eine Verbindung der Formel I nach Anspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der Formel I nach Anspruch 1 und/oder einem ihrer physiologisch unbedenklichen Salze.

6. Verbindungen der Formel I nach Anspruch 1 und ihre physiologisch unbedenklichen Salze zur Bekämpfung von Krankheiten.

7. Verwendung von Verbindungen der Formel I nach Anspruch 1 und/oder ihrer physiologisch unbedenklichen Salze zur Herstellung eines Arzneimittels.

8. Verwendung von Verbindungen der Formel I nach Anspruch 1 und/oder ihrer physiologisch unbedenklichen Salze bei der Bekämpfung von Krankheiten.
